# EUROPÄISCHE PATENTSCHRIFT

(11) **EP 2 730 916 B1**
(45) Veröffentlichungstag und Bekanntmachung des Hinweises auf die Patenterteilung: **13.07.2016**
(21) Anmeldenummer: 13004644.4
(22) Anmeldetag: 25.09.2013
(51) Int. Cl.: G01N 27/417, G01N 33/00

(54) **Verfahren und Vorrichtung zur Kalibrierung eines Abgassensors**
Method and device for calibrating an exhaust gas sensor
Procédé et dispositif d'étalonnage d'un capteur de gaz d'échappement

(30) Priorität: 09.11.2012 DE 102012021928
(43) Veröffentlichungstag der Anmeldung: 14.05.2014
(73) Patentinhaber: MAN Truck & Bus AG, 80995 München (DE)
(72) Erfinder: Döring, Andreas, 82008 München (DE)

(56) Entgegenhaltungen:
- WO-A1-97/31265
- DE-A1- 4 308 191

## Beschreibung

Die Erfindung betrifft ein Verfahren zur Kalibrierung eines Abgassensors gemäß dem Oberbegriff des Patentanspruchs 1 sowie eine Vorrichtung zum Kalibrieren eines Abgassensors nach dem Patentanspruch 8.

Zur Regelung des Betriebs von Brennkraftmaschinen werden Abgassensoren verwendet, die Messsignale an die Motorsteuerung und/oder an ein Abgasnachbehandlungssystem liefern. Als Abgassensoren werden dabei vorwiegend Lambdasonden und NOₓ-Sensoren verwendet. Um eine effektive katalytische Reduktion, beispielsweise mit gezielter Zugabe von Harnstoff, durchführen zu können, ist es erforderlich, mittels NOₓ-Sensoren eine Bestimmung der NOₓ-Emissionen im Abgasstrom einer Brennkraftmaschine vorzunehmen. In der DE 101 00 420 A1 ist ein Verfahren zur Steuerung eines Abgasnachbehandlungssystems beschrieben, bei dem abhängig vom Zustand der Brennkraftmaschine und/oder des Abgasnachbehandlungssystems eine vorgebbare Menge an Reduktionsmittel dem Abgasstrom zugeführt wird.

Zur Messung des Sauerstoffgehalts im Abgasstrom einer Brennkraftmaschine werden Lambdasensoren eingesetzt, die vor und/oder nach einem Katalysator am Abgassystem als Abgassensoren eingesetzt werden.

Die verwendeten Abgassensoren unterliegen einem Alterungsprozess, der dazu führt, dass sich die Messkennlinie während einer längeren Betriebsdauer eines Abgassensors in Abhängigkeit von der Betriebsdauer verändert. Um eine Kalibrierung einer Lambdasonde vorzunehmen, ist es bei Fahrzeugmotoren grundsätzlich bekannt, die Lambdasonden im Schubbetrieb, bei dem kein Abgas anfällt und angesaugte Umgebungsluft die Lambdasonde beaufschlagt, eine Kalibrierung vorzunehmen. Unter der Voraussetzung, dass die angesaugte Umgebungsluft einen Sauerstoffgehalt von 20,942 % hat, kann ein von der Lambdasonde abgegebener Messwert entsprechend korrigiert werden, wenn die Lambdasonde bereits aufgrund längerer Betriebsdauer einen vom tatsächlichen Wert abweichenden Messwert liefert. Eine solche Messwertanpassung kann beispielsweise durch eine Beaufschlagung der von der Lambdasonde abgegebenen Messwerte mit einem Korrekturfaktor erfolgen.

Aus der DE 10 2008 046 121 A1 ist ein Verfahren zum Kalibrieren eines von einem im einer Abgasleitung einer Brennkraftmaschine angeordneten Abgassensors bekannt, der seitlich in einen Abgaskanal ragt. Zum Kalibrieren des Abgassensors wird dieser mit vorbeiströmender Spülluft an seiner Messspitze beaufschlagt. Außerdem wird die Möglichkeit erwähnt, dass eine Abschirmvorrichtung an die Messspitze geführt werden kann, um Spülluft zielgenau an die Messspitze zu leiten. Im normalen Betriebszustand wird die Abschirmvorrichtung aus der Abgasleitung entfernt.

WO97/31265 beschreibt eine Vorrichtung zum Steuern der Verdünnung einer Abgasprobe aus der Abgasanlage eines Motors zur Analyse durch eine Abgasschadstoffanalysevorrichtung.

DE4308191 lehrt ein Gasanalysegerät wobei Faktoren die zu Turbulenzen und Konvektion von Probengas führen bei der Probengas-Zuführleitung mit der Probengas in einen Gasanalysator eingeleitet wird vermieden werden. Die Abgas-Einlaßöffnung ist in die Tiefe der Kalibriergas-Auslassöffnung hineinversetzt, so dass beim Prozeß des Einleitens von Kalibriergas der Umfang der Abgas-Einlaßöffnung des Gaseinlaßrohrs durch das Kalibriergas abgeschirmt wird und nur das Kalibriergas ohne Abgas zugeführt werden kann.

Der Erfindung liegt die Aufgabe zugrunde, ein Verfahren zur Kalibrierung eines Abgassensors anzugeben, mit dem eine möglichst schnell und genau durchführbare Kalibrierung möglich ist.

Die Lösung dieser Aufgabe wird durch die im Patentanspruch 1 angegebenen Merkmale erhalten. Besonders vorteilhafte Weiterbildungen des erfindungsgemäßen Verfahrens sind in den darauf rückbezogenen Unteransprüchen offenbart.

Gemäß den kennzeichnenden Merkmalen des Patentanspruchs 1 ist vorgesehen, dass zu Beginn einer Kalibrierungsphase das in einem Messraum befindliche Abgas, in welchem sich der Abgassensor befindet, durch eine Befüllung des Messraums mit Kalibriergas verdrängt wird, und dass nach Beendigung der Kalibrierphase Abgas in den Messraum diffundiert und/oder eingeleitet wird. Durch eine gezielte Einleitung von Kalibriergas mit einem entsprechenden Druck, kann das vor einer Kalibrierphase im Messraum befindliche Abgas schnell aus diesem verdrängt werden. Bevorzugt wird dann während der Kalibrierphase weiterhin wenigstens eine geringe Menge an Kalibriergas in den Messraum eingeleitet, um dadurch sicherzustellen, dass kein Abgas in den Messraum eindringen kann. Nach Beendigung der Kalibrierphase wird wiederum Abgas direkt in den Messraum eingeleitet, wodurch darin befindliches Kalibriergas aus dem Messraum entweicht und sehr schnell wieder der normale Messbetrieb aufgenommen werden kann. Als Kalibriergas kann grundsätzlich jedes hierfür geeignete Gas verwendet werden. Besonders bevorzugt ist eine Ausführungsvariante, bei der in Verbindung mit aufgeladenen Brennkraftmaschinen als Kalibriergas komprimierte Frischluft von wenigstens einem Verdichter verwendet wird.

Während der Kalibrierphase kann es auch ausreichend sein, wenn der Messraum nicht vollständig vom Abgasstrom abgeschirmt bzw. abgetrennt ist, wie dies beispielsweise bei der Verwendung von Ventilen der Fall wäre, wenn durch ständig in den Messraum zugeführtes Kalibriergas dabei sichergestellt ist, dass kein Abgas in den Messraum eindringen kann. Eine nicht vollständige Abschirmung des Abgasstroms kann beispielsweise dadurch gegeben sein, dass der Messraum über eine gasdurchlässige Membran vom Abgasstrom abgeschirmt ist oder die in den Messraum zugeführte Kalibiergasmenge und/oder der im Messraum vorherrschende Druck so groß sind, dass kein Abgas in den Messraum eindringen kann.

Besonders vorteilhaft ist eine Ausführungsform, bei der nach Beendigung der Kalibrierphase Abgas in den Messraum gesaugt und/oder gedrückt wird, um den Messraum schnell zu spülen. Hierzu kann Abgas nach Beendigung der Kalibrierphase mittels einer Ansaugvorrichtung in den Messraum gesaugt werden. Alternativ oder aber auch zusätzlich dazu kann vorgesehen sein, dass der Messraum nach Beendigung der Kalibrierphase strömungstechnisch mit einem Teil der Abgasanlage verbunden wird, in dem ein höherer Druck als im Messraum herrscht, so dass das Abgas in den Messraum gedrückt wird.

Der Erfindung liegt die weitere Aufgabe zugrunde, eine Vorrichtung zum Kalibrieren eines Abgassensors zu schaffen, mit der eine zuverlässige und möglichst schnell durchführbare Kalibrierung möglich ist.

Die Lösung dieser auf die Vorrichtung bezogenen Aufgabe erhält man mit den im Patentanspruch 8 angegebenen Merkmalen. Besonders vorteilhafte Weiterbildungen der erfindungsgemäßen Vorrichtung sind in den darauf rückbezogenen Unteransprüchen.

Gemäß den Merkmalen des Patentanspruchs 8 liegt der Abgassensor in einem Messraum ein, der gegenüber dem im Abgaskanal vorhandenen Abgasstrom mittels einer wenigstens zeitweise gasdurchlässigen Abschirmvorrichtung abgeschirmt ist. Über eine an den Messraum angeschlossene Gasleitung kann Kalibriergas in den Messraum eingeleitet werden. Durch die Anordnung des Abgassensors in einem Messraum, der im Abgaskanal oder unmittelbar angrenzend an den Abgaskanal angeordnet sein kann, kann ein gezielter Gasaustausch im Messraum vorgenommen werden, um beispielsweise sehr schnell die Kalibrierphase einleiten zu können. Über eine Gasleitung kann mit entsprechendem Druck Kalibriergas in den Messraum eingespeist werden, wodurch eine schnelle Verdrängung des im Messraum zuvor befindlichen Abgases erreicht wird. Damit das Kalibriergas in den Messraum einleitbar ist, ist es erforderlich, dass die Abschirmvorrichtung wenigstens zeitweise gasdurchlässig ist.

Als zeitweise gasdurchlässige Abschirmvorrichtung können zwei ineinander konzentrisch angeordnete, perforierte Zylinder verwendet werden, von denen wenigstens einer um seine Längsachse zur Veränderung der Abschirmwirkung verstellbar ist. Durch eine Drehung um seine Längsachse können die Perforierungen der Zylinder in eine fluchtende Position oder in eine nicht fluchtende Position gebracht werden. In einer fluchtenden Position sind die Zylinderseitenwände für den Abgasstrom durchlässig, so dass dieser durch den Messraum hindurchströmen kann. Werden dagegen die Zylinder so verdreht, dass die Perforierungen nicht fluchten, ergibt sich dadurch eine zumindest weitgehend dichte Abschirmung gegenüber dem Abgasstrom. Der Messraum kann dann mit Kalibriergas gefüllt werden, um eine Kalibrierung des im Messraum angeordneten Abgassensors durchführen zu können.

Die Perforierungen können in den Zylinderwänden als um jeweils 180 Grad versetzt angeordnete Bohrungen ausgebildet sein. Dadurch erhält man einen von den konzentrisch angeordneten Zylindern gebildeten Messraum, der einen verhältnismäßig einfachen und funktionssicheren Aufbau hat.

Der Messraum kann auch an den Abgaskanal einer Brennkraftmaschine unmittelbar angrenzend angeordnet sein, wobei über eine gasdurchlässige Membran, die eine Abschirmvorrichtung bildet, Gas in und aus dem Messraum herausgeführt werden kann. Über eine an den Messraum angeschlossene Gasleitung kann Kalibriergas in den Messraum eingeleitet werden, wobei im Messraum befindliches Abgas durch die gasdurchlässige Membran in den Abgaskanal verdrängt wird. Während der Kalibrierphase kann dann ständig eine gewisse Menge an Kalibriergas in den Messraum geleitet werden, um sicherzustellen, dass durch die gasdurchlässige Membran kein Abgas in den Messraum eindringt. Bei Beendigung der Kalibrierphase wird die Zufuhr von Kalibriergas gestoppt, so dass über die gasdurchlässige Membran wieder Abgas in den Messraum eindringen kann.

Um ein beschleunigtes Eindringen von Abgas in den Messraum zu bewirken, kann auch über eine an den Messraum angeschlossene Saugleitung ein Ansaugen des Abgases aus dem Abgaskanal durch die gasdurchlässige Membran erfolgen. Der Ansaugvorgang kann dabei mittels einer steuerbaren Ansaugvorrichtung durchgeführt werden, die beispielsweise aus einer ohnehin vorhandenen Saugleitung eines Motoransaugsystems und einem steuerbaren Ventil in einer zum Messraum führenden Saugleitung bestehen kann. Alternativ, aber ggf. auch zusätzlich dazu, kann vorgesehen sein, dass der Messraum strömungstechnisch mit einem Teil der Abgasanlage verbunden ist, in dem ein höherer Druck als im Messraum herrscht, so dass das Abgas in den Messraum gedrückt wird. Bevorzugt wird hier das Abgas stromauf einer Abgasturbine eines Abgasturboladers und/oder stromauf einer Drosseleinrichtung und/oder stromauf eines Schalldämpfers abgezweigt.

Die Erfindung wird nachfolgend anhand von in der Zeichnung dargestellten Ausführungsbeispielen näher erläutert.

Es zeigen:
- Fig. 1: eine schematische Darstellung einer Brennkraftmaschine mit einem Abgasnachbehandlungssystem, an dessen Abgaskanal ein Abgassensor angeschlossen ist,
- Fig. 2: eine Anordnung eines Abgassensors in einem sich an einen Abgaskanal anschließenden Messraum,
- Fig. 3: einen in einem Abgaskanal gebildeten Messraum mit einem Abgassensor,
- Fig. 4: eine Schnittansicht entlang der Schnittlinie AA im Bereich des Messraums von Fig. 3, der von zwei ineinander angeordneten Zylindern gebildet wird,
- Fig. 5: eine Schnittansicht entlang der Schnittlinie AA wie bei Fig. 4, jedoch mit einem um 90 Grad gedrehten inneren Zylinder, und
- Fig. 6: eine alternative Ausgestaltung zur Ausführungsform gemäß Fig. 1, mit der nach Beendigung der Kalibrierphase Abgas in den Messraum eingedrückt werden kann.

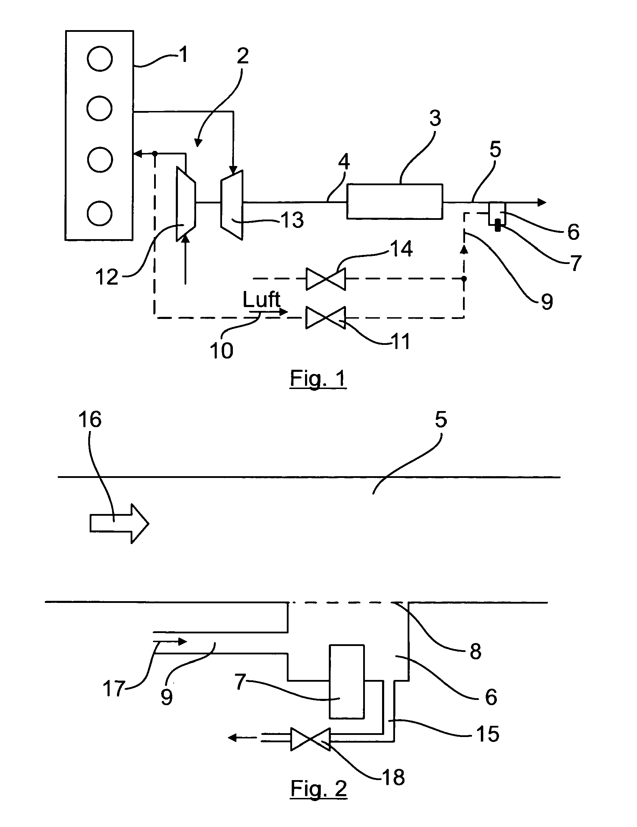

In Fig. 1 ist eine Brennkraftmaschine 1 mit einem Abgasturbolader 2 dargestellt, an den eine zu einem Katalysator 3 führender Abgaskanal 4 angeschlossen ist. Stromab des Katalysators 3 befindet sich ein Abgaskanal 5, an den ein Messraum 6 mit einem Abgassensor 7 angrenzt.

Der Messraum 6 ist wie in Fig. 2 dargestellt strömungstechnisch mit dem Abgaskanal 5 über eine gasdurchlässige Membran 8 (Fig. 2) verbunden. Um Kalibriergas in den Messraum 6 einleiten zu können, ist an den Messraum 6 eine Gasleitung 9 angeschlossen, über die beispielsweise Luft als Kalibriergas gemäß der Pfeilrichtung 10 über ein steuerbares Ventil 11 eingespeist werden kann. Die Luft wird ausgangsseitig eines zum Turbolader 2 gehörenden Verdichters 12 abgezweigt und gelangt bei geöffnetem Ventil 11 in den Messraum 6. Der Verdichter 12 dient hier als Druckerzeuger und ist Teil des Abgasturboladers 2, der vom Abgas der Brennkraftmaschine 1 über die Turbine 13 des Turboladers 2 angetrieben wird.

Das vom Verdichter dem Messraum 6 zugeführte Kalibriergas, beispielsweise Luft, kann bei Verwendung eines Lambdasensors zu dessen Kalibrierung verwendet werden, während es bei anderen Sensoren, wie beispielsweise NOx-, NH3- oder Russsensoren, zur Bestimmung des Nullpunkts herangezogen werden kann.

Im Ausführungsbeispiel von Fig. 1 kann über ein zweites steuerbares Ventil 14 ein weiteres Kalibriergas mit einer vorgegebenen Zusammensetzung über die Gasleitung 9 in den Messraum 6 eingespeist werden. In diesem Fall ist das Ventil 11 geschlossen. Das Kalibriergas besitzt zum Beispiel eine vorgegebene NOₓ-Konzentration, wodurch beispielsweise ein Korrekturfaktor für einen als NOₓ-Sensor ausgebildeten Abgassensor ermittelt werden kann, wenn der vom Abgassensor ermittelte Messwert von dem tatsächlichen Wert der eingespeisten NOₓ-Konzentration abweicht.

In Fig. 2 ist der Bereich, wo der Messraum 6 an den Abgaskanal 5 angrenzt, vergrößert dargestellt. In den Messraum 6 ragt ein mit einem nicht näher dargestellten Messsystem elektrisch verbundener Abgassensor 7 hinein. Außerdem sind an den Messraum 6 die in Fig. 1 dargestellte Gasleitung 9 und eine weitere Saugleitung 15 angeschlossen. Der Messraum 6 ist durch eine Abschirmvorrichtung bildende gasdurchlässige Membran 8 vom Abgasstrom, der in Pfeilrichtung 16 den Abgaskanal 5 durchströmt, teilweise abgeschirmt.

Wird in den Messraum 6 über die Gasleitung 9 Luft bzw. ein sonstiges Kalibriergas gemäß der Pfeilrichtung 17 eingespeist, wobei die Saugleitung 15 durch ein geschlossenes Ventil 18 gesperrt ist, hat dies zur Folge, dass vorhandenes Abgas aus dem Messraum 6 durch die Membran 8 in den Abgaskanal 5 verdrängt wird. Im Messraum 6 befindet sich dann nur noch Luft bzw. Kalibriergas, so dass eine Kalibriermessung durchgeführt werden kann.

Zur Beendigung der Kalibrierphase wird im dargestellten Ausführungsbeispiel gemäß Fig. 2 über die Saugleitung 15 bei geöffnetem Ventil 18 Abgas in den Messraum 6 durch die Membran 8 angesaugt. Die Gasleitung 9 ist dabei gesperrt. Durch die Ansaugung von Abgas in den Messraum 6 wird erreicht, dass der Abgassensor 7 schnell vollständig mit Abgas beaufschlagt wird und der Abgassensor wieder im normalen Messbetrieb zur Abgasmessung verwendet werden kann.

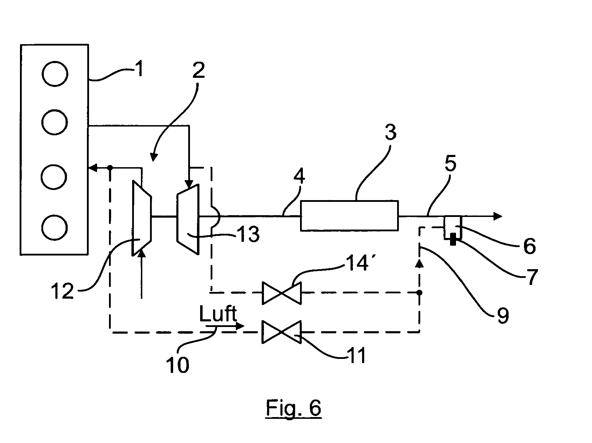

Die Ausführungsform gemäß Fig. 6 zeigt eine zur Ausführungsform nach Fig. 1 alternative oder ggf. auch zusätzliche Möglichkeit, den Messraum mit Abgas zu füllen, bei der Abgas nach Beendigung der Kalibrierphase in den Messraum eingedrückt wird. Hierfür ist es vorteilhaft, Abgas an einer Stelle zu entnehmen, an dem ein hoher Abgasgegendruck vorherrscht, so dass das Abgas auf Grund der Druckdifferenz in den Messraum gedrückt wird. Bei abgasaufgeladenen Brennkraftmaschinen 1 bietet es sich somit an, das Abgas stromauf der Abgasturbine 13 zu entnehmen, da hier ein deutlich höherer Druck als stromab der Abgasturbine 13 vorliegt. Die Entnahme wird vorzugsweise mittels eines steuerbaren Ventils 14' gesteuert bzw. geregelt. Aber auch eine Entnahme stromauf von Drosselstellen oder gegendruckerhöhenden Einbauten, wie Schalldämpfern, Katalysatoren oder Drosselklappen ist grundsätzlich möglich und denkbar.

Dauer und Häufigkeit der Kalibrierphasen können je nach Betriebszustand der Brennkraftmaschine und/oder des Abgasnachbehandlungssystems eingestellt bzw. verändert werden.

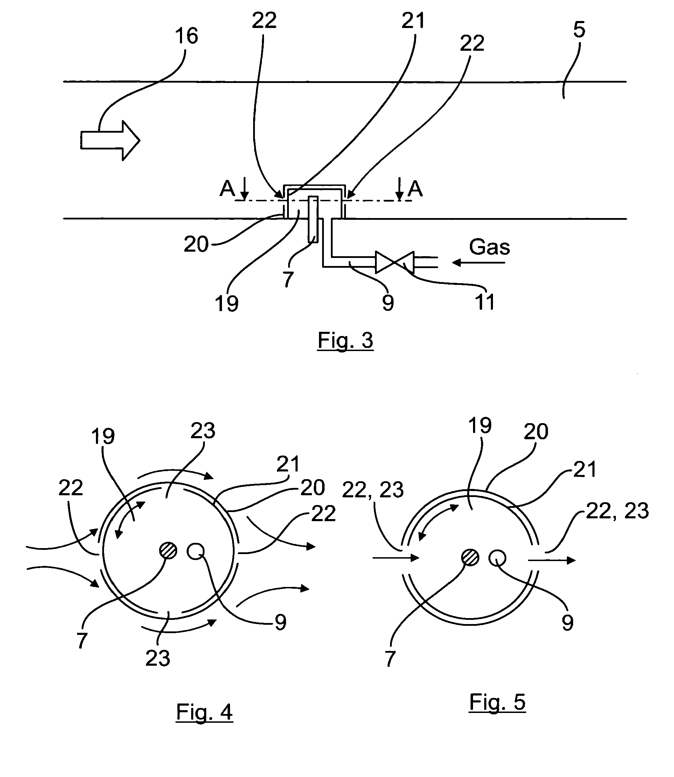

Fig. 3 zeigt eine bevorzugte Ausführung eines Messraums 19, der in einem Abgaskanal 5 angeordnet ist und von zwei konzentrisch ineinander angeordneten perforierten Zylindern 20, 21 gebildet wird. Der Abgassensor 7 ragt in den Messraum 19. Eine Gasleitung 9 ist an den Messraum 19 angeschlossen, über die Luft oder Spülgas oder Kalibriergas in den Messraum 19 geleitet werden kann. Die Gasleitung 9 ist mittels eines steuerbaren Ventils 11 absperrbar.

Der äußere Zylinder 20 und der innere Zylinder 21 besitzen im dargestellten Ausführungsbeispiel jeweils um 180 Grad versetzt angeordnete Bohrungen 22, 23 (Fig. 4). In Fig. 3 sind diese Bohrungen 22, 23, wie in der Schnittansicht von Fig. 4, nicht fluchtend angeordnet, so dass der Messraum 19 für den Abgasstrom gesperrt ist. Der innere Zylinder 21 ist jedoch gemäß dargestelltem Doppelpfeil um 90 Grad in eine Position drehbar, die in Fig. 5 dargestellt ist. In dieser Position fluchten die Bohrungen 22, 23, so dass ein Teil des Abgasstroms in und durch den Messraum 19 strömen kann. In der Position gemäß Fig. 5 ist der Messraum 19 für den Abgasstrom offen, so dass der Abgassensor 7 eine Abgasmessung bezüglich des Sauerstoffanteils oder der NOₓ-Konzentration vornehmen kann. Befindet sich dagegen der innere Zylinder 21 in der Position, wie sie in Fig. 4 dargestellt ist, befindet sich das System in einer Kalibrierphase, in der Kalibriergas über die Gasleitung 9 in den Messraum 19 eingeführt werden kann.

An den Messraum 19 kann eine hier nicht dargestellte weitere Saugleitung angeschlossen sein, um bei geschlossenem Messraum 19 über die Gasleitung 9 ungehindert Gas einführen zu können. Die Zylinder 20, 21 können aber auch gasdurchlässige Bereiche besitzen, um die Einleitung von Gas beschleunigt durchführen zu können.

## Patentansprüche

1. Verfahren zur Kalibrierung eines Abgassensors (7), der in einem Messraum (6) angeordnet ist, wobei zu Beginn einer Kalibrierphase im Messraum (6) befindliches Abgas durch eine Befüllung des Messraums (6) mit Kalibriergas verdrängt wird, und dass bei Beendigung der Kalibrierphase Abgas in den Messraum (6) diffundiert und/oder eingeleitet wird **dadurch gekennzeichnet, dass**, der Messraum (6) sich im Abgaskanal (5) einer Brennkraftmaschine (1) befindet und aus zwei ineinander konzentrisch angeordneten, perforierten Zylindern (20, 21) besteht, von denen wenigstens einer um seine Längsachse verstellbar ist, so dass beide Zylinder (20, 21) eine Abschirmvorrichtung mit veränderbarer Abschirmwirkung bilden und dass die Zylinder (20, 21) als Perforierung jeweils mindestens zwei, insbesondere um 180 Grad versetzt angeordnete Bohrungen (22, 23) in den Zylinderwänden haben, die aus einer nicht fluchtenden Position in eine fluchtende Position zur Öffnung des Messraums (6) für einströmendes Abgas verstellt werden.

2. Verfahren nach Anspruch 1, **dadurch gekennzeichnet, dass** während der gesamten Kalibrierphase Kalibriergas in den Messraum (6) eingeleitet wird, so dass im Messraum (6) ein Überdruck gegenüber dem im Abgaskanal (5) vorhandenen Druck aufrecht erhalten wird.

3. Verfahren nach Anspruch 2, **dadurch gekennzeichnet, dass** während der Kalibrierphase der Messraum (6) vom Abgasstrom im Abgaskanal (5) nicht vollständig abgeschirmt ist.

4. Verfahren nach einem der vorhergehenden Ansprüche, **dadurch gekennzeichnet, dass** nach Beendigung der Kalibrierphase Abgas in den Messraum (6) gesaugt und/oder gedrückt wird.

5. Verfahren nach Anspruch 4, **dadurch gekennzeichnet, dass** das Abgas nach Beendigung der Kalibrierphase mittels einer Ansaugvorrichtung in den Messraum (6) gesaugt wird.

6. Verfahren nach Anspruch 4 oder 5, **dadurch gekennzeichnet, dass** der Messraum (6) nach Beendigung der Kalibrierphase strömungstechnisch mit einem Teil der Abgasanlage verbunden wird, in dem ein höherer Druck als im Messraum (6) herrscht, so dass das Abgas in den Messraum (6) gedrückt wird.

7. Verfahren nach einem der vorangegangenen Ansprüche, **dadurch gekennzeichnet, dass** bei aufgeladenen Brennkraftmaschinen als Kalibriergas komprimierte Frischluft von wenigstens einem Verdichter verwendet wird.

8. Vorrichtung zum Kalibrieren eines Abgassensors (7), insbesondere einer Lambdasonde und/oder eines NOₓ-Sensors, der an einem Abgaskanal (5) eines Abgassystems einer Brennkraftmaschine (1) angeordnet ist und mittels einer Abschirmvorrichtung vom Abgas abschirmbar und über eine Gasleitung (9) mit einem Kalibriergas beaufschlagbar ist, der Abgassensor (7) in einem Messraum (6) einliegt, dass der Messraum (6) gegenüber dem im Abgaskanal (5) vorhandenen Abgasstrom mittels einer wenigstens zeitweise gasdurchlässigen Abschirmvorrichtung (8; 20, 21) abgeschirmt ist, und dass die Gasleitung (9) an den Messraum (6) angeschlossen ist **dadurch gekennzeichnet, dass**,
• der Messraum (6) sich im Abgaskanal (5) befindet und aus zwei ineinander konzentrisch angeordneten, perforierten Zylindern (20, 21) besteht, von denen wenigstens einer um seine Längsachse verstellbar ist, so dass beide Zylinder (20, 21) eine Abschirmvorrichtung mit veränderbarer Abschirmwirkung bilden oder
• der Messraum (6) unmittelbar an den Abgaskanal (5) angrenzt und gegenüber dem Abgasstrom durch eine als Abschirmvorrichtung dienende gasdurchlässige Membran (8) abgeschirmt ist.

9. Vorrichtung nach Anspruch 8, **dadurch gekennzeichnet, dass** die Zylinder (20, 21) als Perforierung jeweils mindestens zwei, insbesondere um 180 Grad versetzt angeordnete Bohrungen (22, 23) in den Zylinderwänden haben, die aus einer nicht fluchtenden Position in eine fluchtende Position zur Öffnung des Messraums (6) für einströmendes Abgas verstellbar sind.

10. Vorrichtung nach einem der Ansprüche 8 oder 9, **dadurch gekennzeichnet, dass** an den Messraum (6) eine steuerbare Ansaugvorrichtung angeschlossen ist, mittels der das Abgas nach Beendigung der Kalibrierphase mittels einer Ansaugvorrichtung in den Messraum (6) saugbar ist.

11. Vorrichtung nach Anspruch 10, **dadurch gekennzeichnet, dass** die Ansaugvorrichtung eine Saugleitung (15) und ein steuerbares Ventil (18) umfasst.

12. Vorrichtung nach einem der Ansprüche 8 bis 11, **dadurch gekennzeichnet, dass** der Messraum (6) strömungstechnisch mit einem Teil der Abgasanlage verbunden ist, in dem ein höherer Druck als im Messraum (6) herrscht, so dass das Abgas in den Messraum (6) gedrückt wird.

13. Vorrichtung nach Anspruch 12, **dadurch gekennzeichnet, dass** das Abgas stromauf einer Abgasturbine (13) eines Abgasturboladers (2) und/oder stromauf einer Drosseleinrichtung und/oder stromauf eines Schalldämpfers abgezweigt ist.

14. Fahrzeug, insbesondere Nutzfahrzeug, mit einer Vorrichtung nach einem der vorhergehenden Ansprüche 8 bis 13 zur Durchführung eines Verfahrens nach einem der Ansprüche 1 bis 7.

## Claims

1. A method for calibrating an exhaust gas sensor (7) which is arranged in a measurement chamber (6), wherein at the start of a calibration phase, exhaust gas present in the measurement chamber (6) is displaced by a filling of the measurement chamber (6) with calibration gas, and that at the end of the calibration phase, exhaust gas is diffused and/or introduced into the measurement chamber (6), **characterized in that** the measurement chamber (6) is in the exhaust channel (5) of an internal combustion engine (1) and consists of two perforated cylinders (20, 21) arranged concentrically one inside the other, of which at least one is movable about its longitudinal axis so that the two cylinders (20, 21) form a screening device with variable screening effect and **in that** as perforations the cylinders (20, 21) each have at least two bores (22, 23), in particular arranged offset by 180°, in the cylinder walls which are moved from a non-aligned position into an aligned position to open the measurement chamber (6) to inflowing exhaust gas.

2. The method according to Claim 1, **characterized in that** calibration gas is introduced into the measurement chamber (6) throughout the entire calibration phase so that a higher pressure is maintained in the measurement chamber (6) than the pressure predominating in the exhaust channel (5).

3. The method according to Claim 2, **characterized in that** during the calibration phase, the measurement chamber (6) is not completely screened from the exhaust gas flow in the exhaust channel (5).

4. The method according to any of the preceding claims, **characterized in that** after the end of the calibration phase, exhaust gas is drawn and/or pressed into the measurement chamber (6).

5. The method according to Claim 4, **characterized in that** after the end of the calibration phase, the exhaust gas is drawn into the measurement chamber (6) by means of a suction device.

6. The method according to Claim 4 or 5, **characterized in that** after the end of the calibration phase, the measurement chamber (6) is connected fluidically to a part of the exhaust system in which a higher pressure predominates than in the measurement chamber (6), so that the exhaust gas is pressed into the measurement chamber (6).

7. The method according to any of the preceding claims, **characterized in that** in the case of charged internal combustion engines, compressed fresh air from at least one compressor is used as a calibration gas.

8. A device for calibrating an exhaust gas sensor (7), in particular a lambda sensor and/or an NOₓ sensor, which is arranged on an exhaust channel (5) of an exhaust system of an internal combustion engine (1) and which can be screened from the exhaust gas by means of a screening device and which can be exposed to a calibration gas via a gas pipe (9), the exhaust gas sensor (7) lies in a measurement chamber (6), that the measurement chamber (6) is screened from the exhaust gas flow present in the exhaust channel (5) by means of a screening device (8; 20, 21) which is gas-permeable for at least part of the time, and that the gas pipe (9) is connected to the measurement chamber (6), **characterized in that**
• the measurement chamber (6) is in the exhaust channel (5) and consists of two perforated cylinders (20, 21) arranged concentrically one inside the other, of which at least one is movable about its longitudinal axis so that the two cylinders (20, 21) form a screening device with variable screening effect or
• the measurement chamber (6) is directly adjacent to the exhaust channel (5) and is screened from the exhaust gas flow by a gas-permeable membrane (8) serving as a screening device.

9. The device according to Claim 8, **characterized in that** as perforations the cylinders (20, 21) each have at least two bores (22, 23), in particular arranged offset by 180°, in the cylinder walls which are movable from a non-aligned position into an aligned position to open the measurement chamber (6) to inflowing exhaust gas.

10. The device according to either of Claims 8 and 9, **characterized in that** a controllable suction device is connected to the measurement chamber (6), by means of which, after the end of the calibration phase, the exhaust gas can be drawn into the measurement chamber (6) by means of a suction device.

11. The device according to Claim 10, **characterized in that** the suction device comprises a suction line (15) and a controllable valve (18).

12. The device according to any of Claims 8 to 11, **characterized in that** the measurement chamber (6) is connected fluidically to a part of the exhaust system in which a higher pressure predominates than in the measurement chamber (6), so that the exhaust gas is pressed into the measurement chamber (6).

13. The device according to Claim 12, **characterized in that** the exhaust gas is extracted upstream of an exhaust gas turbine (13) of an exhaust turbocharger (2) and/or upstream of a choke device and/or upstream of a silencer.

14. A vehicle, in particular a utility vehicle, with a device according to any of the preceding Claims 8 to 13 for performance of a method according to any of Claims 1 to 7.

## Revendications

1. Procédé d'étalonnage d'un capteur de gaz d'échappement (7) disposé dans une chambre de mesure (6), au début d'une phase d'étalonnage, le gaz d'échappement se trouvant dans la chambre de mesure (6) étant refoulé par un remplissage de la chambre de mesure (6) en gaz d'étalonnage et à la fin de la phase d'étalonnage, le gaz d'échappement étant diffusé et/ou amené dans la chambre de mesure (6), **caractérisé en ce que** la chambre de mesure (6) se trouve dans le canal de gaz d'échappement (5) d'un moteur à combustion interne (1) et se compose de deux cylindres (20, 21) perforés disposés concentriquement l'un dans l'autre parmi lesquels au moins un peut être déplacé autour de son axe longitudinal, de sorte que les deux cylindres (20, 21) forment un dispositif de protection avec effet de protection variable et que les cylindres (20, 21) ont comme perforation respectivement au moins deux alésages (22, 23) disposés de façon décalée, notamment de 180 degrés, dans les parois de cylindre pouvant être déplacés d'une position non alignée dans une position alignée pour l'ouverture de la chambre de mesure (6) pour le gaz d'échappement entrant.

2. Procédé selon la revendication 1, **caractérisé en ce que** pendant la phase d'étalonnage totale, le gaz d'étalonnage est amené dans la chambre de mesure (6), de sorte qu'une surpression est maintenue par rapport à la pression présente dans le canal de gaz d'échappement (5), dans la chambre de mesure (6).

3. Procédé selon la revendication 2, **caractérisé en ce que** pendant la phase d'étalonnage, la chambre de mesure (6) n'est pas entièrement protégée du flux de gaz d'échappement dans le canal de gaz d'échappement (5).

4. Procédé selon l'une quelconque des revendications précédentes, **caractérisé en ce que** le gaz d'échappement est aspiré et/ou comprimé dans la chambre de mesure (6) à la fin de la phase d'étalonnage.

5. Procédé selon la revendication 4, **caractérisé en ce que** le gaz d'échappement est aspiré à la fin de la phase d'étalonnage dans la chambre de mesure (6) au moyen d'un dispositif d'aspiration.

6. Procédé selon la revendication 4 ou 5, **caractérisé en ce que** la chambre de mesure (6) est reliée, à la fin de la phase d'étalonnage, dans la technique de flux, à une partie de l'installation de gaz d'échappement dans laquelle une pression supérieure règne dans la chambre de mesure (6), de sorte que le gaz d'échappement est comprimé dans la chambre de mesure (6).

7. Procédé selon l'une quelconque des revendications précédentes, **caractérisé en ce que** dans les moteurs à combustion interne suralimentés, l'air frais comprimé provenant d'au moins un compresseur sert de gaz d'étalonnage.

8. Dispositif d'étalonnage d'un capteur de gaz d'échappement (7), notamment d'une sonde lambda et/ou d'un capteur NOₓ, disposé au niveau d'un canal de gaz d'échappement (5) d'un système d'échappement d'un moteur à combustion interne (1) et pouvant être protégé du gaz d'échappement à l'aide d'un dispositif de protection et pouvant être alimenté en gaz d'étalonnage via une conduite de gaz (9), le capteur de gaz d'échappement (7) reposant dans une chambre de mesure (6), que la chambre de mesure (6) est protégée par rapport au flux de gaz d'échappement présent dans le canal de gaz d'échappement (5) à l'aide d'un dispositif de protection (8 ; 20, 21) perméable au gaz au moins temporairement et que la conduite de gaz (9) est raccordée à la chambre de mesure (6), **caractérisé en ce que** :
- la chambre de mesure (6) se trouve dans le canal de gaz d'échappement (5) et se compose de deux cylindres (20, 21) perforés disposés concentriquement l'un dans l'autre, parmi lesquels au moins un peut être déplacé autour de son axe longitudinal, de sorte que les deux cylindres (20, 21) forment un dispositif de protection avec effet de protection variable ; ou
- la chambre de mesure (6) est délimitée directement au niveau du canal de gaz d'échappement (5) et protégée par rapport au flux de gaz d'échappement par une membrane (8) perméable au gaz servant de dispositif de protection.

9. Dispositif selon la revendication 8, **caractérisé en ce que** les cylindres (20, 21) ont comme perforation respectivement au moins deux alésages (22, 23) disposés de façon décalée, notamment de 180 degrés, dans les parois de cylindre et pouvant être déplacés d'une position non alignée dans une position alignée pour l'ouverture de la chambre de mesure (6) pour le gaz d'échappement entrant.

10. Dispositif selon l'une quelconque des revendications 8 ou 9, **caractérisé en ce qu'**un dispositif d'aspiration commandable est raccordé à la chambre de mesure (6), le gaz d'échappement pouvant être aspiré dans la chambre de mesure (6) à la fin de la phase d'étalonnage au moyen d'un dispositif d'aspiration à l'aide dudit dispositif.

11. Dispositif selon la revendication 10, **caractérisé en ce que** le dispositif d'aspiration comprend une conduite d'aspiration (15) et une soupape (18) commandable.

12. Dispositif selon l'une quelconque des revendications 8 à 11, **caractérisé en ce que** la chambre de mesure (6) est reliée, à la fin de la phase d'étalonnage, dans la technique de flux, à une partie de l'installation de gaz d'échappement dans laquelle une pression supérieure règne dans la chambre de mesure (6), de sorte que le gaz d'échappement est comprimé dans la chambre de mesure (6).

13. Dispositif selon la revendication 12, **caractérisé en ce que** le gaz d'échappement est divisé en amont d'une turbine de gaz d'échappement (13) d'un turbocompresseur de gaz d'échappement (2) et/ou en amont d'un dispositif d'étranglement et/ou en amont d'un silencieux.

14. Véhicule, notamment véhicule utilitaire, avec un dispositif selon l'une quelconque des revendications précédentes 8 à 13 pour la mise en oeuvre d'un procédé selon l'une quelconque des revendications 1 à 7.
